# EUROPEAN PATENT SPECIFICATION

(11) **EP 1 643 966 B1**
(45) Date of publication and mention of the grant of the patent: **21.07.2010**
(21) Application number: 04755117.1
(22) Date of filing: 15.06.2004
(51) Int. Cl.: A61K 8/64, A61Q 5/00

(54) **MODIFIED SOY PROTEINS IN SKIN TIGHTENING COMPOSITIONS**
MODIFIZIERTE SOJAPROTEINE IN KOSMETISCHEN ZUSAMMENSETZUNGEN FÜR HAUTSTRAFFUNG
PROTEINES DE SOJA MODIFIEES INTEGREES DANS DES COMPOSITIONS ASTRINGENTES DE SOIN CUTANE

(30) Priority: 17.06.2003 US 479330 P
(43) Date of publication of application: 12.04.2006
(73) Proprietor: Cognis IP Management GmbH, 49589 Düsseldorf (DE)
(72) Inventor: SCHULTZ, Thomas, M., Randolph, NJ 07869 (US); TRAN, Huu, Tam, St. Louis, MO 63021 (US); MAMPE, Dirk, 40476 Düsseldorf (DE)
(74) Representative: Gittinger, Andreas
(86) International application number: PCT/US2004/018767
(87) International publication number: WO 2004/112732

(56) References cited:
- EP-A- 0 455 468
- EP-A- 1 054 103
- EP-A- 1 172 087
- DE-A- 10 230 043
- US-A- 4 474 694
- US-A- 4 554 337
- US-A- 4 824 662

## Description

### CROSS-REFERENCE TO RELATED APPLICATION

This application claims the benefit of U.S. Provisional Application Serial No. 60/479,330, filed on June 17, 2003.

### FIELD OF THE INVENTION

The invention relates to the field of personal care products. More specifically, the invention relates to the use of modified soy proteins in compositions for skin tightening.

### BACKGROUND OF THE INVENTION

Film-forming substances are widely used in compositions for skin care and hair care as conditioning agents and moisturizers, and to protect the skin and hair against environmental and chemical damage. These substances adsorb onto and/or absorb into the skin or hair, forming a protective coating. Commonly used film-forming substances include synthetic polymers, such as silicones, polyvinylpyrrolidone, acrylic acid polymers, and polysaccharides, and proteins, such as collagen, keratin, elastin, silk, and soy proteins. Many proteins are known to be particularly effective film-forming agents, depending on their molecular weight. Because of their low solubility at the conditions used in skin and hair care products, proteins are commonly used in the form of peptides, formed by the hydrolysis of the protein.

In hair care compositions, film-forming substances are used to form a protective film on the surface of the hair to protect it from damage due to grooming and styling, shampooing, and exposure to ultraviolet light and the reactive chemicals commonly used in permanent wave agents, hair coloring products, bleaches, and hair straighteners, which denature the hair keratin protein. Moreover, these film-forming substances improve the elasticity of the hair. Film-forming substances that have been used in hair care products include proteins, such as keratin, collagen, and silk proteins and hydrolysates thereof, and polymeric materials, such as polyacrylates, long chain alkyl quaternized amines, and siloxane polymers. For example, Cannell at al. in U.S. Patent No. 6,013,250 describe a hair care composition for treating hair against chemical and ultraviolet light damage. This composition comprises hydrolyzed protein, having an abundance of sulfur containing amino acids, and divalent cations. Omura et al. in U.S. Patent No. 6,139,851 describe a hair care cosmetic for treating split ends which contains one or more types of silicone derivatives, one or more types of specific polyether modified silicone, and a lower alcohol. The major problem with these compositions is that they lack the required durability required for long-lasting protection.

Film-forming substances are also used in skin care compositions to form a protective film on the skin. These films can serve to lubricate and coat the skin to passively impede the evaporation of moisture and smooth and soften the skin. Commonly used film-forming substances in skin care compositions include hydrolyzed animal and vegetable proteins (Puchalski et al., U.S. Patent No. 4,416,873, El-Menshawy et al., U.S. Patent No. 4,482,537, and Kojima et al., JP 02311412) and silk proteins (Philippe et al., U.S. Patent No. 6,280,747 and Fahnestock et al., copending U.S. Provisional Patent Application No. 60/448952).

The use of hydrolyzed soy proteins as film-forming agents in personal care compositions is well known (See for example Russ et al. U.S. Patent No. 6,299,890, Burns et al. U.S. Patent No. 5,635,168, Simon et al. U.S. Patent No. 6,284,233, and Yoshida et al. U.S. Patent No. 6,036,730). In most of these compositions, the soy proteins are used in an extensively hydrolyzed form due to the low solubility of the intact proteins.

US-A-4 824 662 relates to a nail varnish remover exhibiting reduced skin dehydration due to the presence of hydrolysed soy protein.

The use of some types of chemically modified soy proteins in personal care compositions is also known. For example, Jones et al. in U.S. Patent No. 5,412,074 describe the use of silicone-modified soy proteins and peptides, formed by reaction with an organofunctional silicone reagent, as a film-forming agent in cosmetic formulations. Hale et al. in GB 1,529,841 describe the use of hydrolyzed soy proteins, which are acylated by reaction with an acid anhydride, in detergent compositions. Marsh et al. in CA 1051312 describe soy protein hydrolyzates that are modified by treatment with an oxidizing agent such as hydrogen peroxide and their use in cosmetic or detergent compositions. In that disclosure, the purpose of the peroxide treatment is to bleach the material to a pale color, thereby making it more cosmetically acceptable. Isao et al. in published application JP 2001031697A describe soy protein hydrolyzates that are modified by reaction with an alkyl or alkenyl glycidyl ether and their use in detergent compositions. Additionally, Alain et al. in FR 2707647 describe modified soy proteins formed by coupling an α-hydroxy acid with a soy protein or peptide and their use in cosmetic and pharmaceutical compositions.

Although the soy proteins known in the art provide a conditioning effect in hair and skin care compositions, there is a need for improved skin tightening agents, preferably for human skin.

Applicants have met the stated need by unexpectedly discovering that certain modified soy proteins, originally developed for industrial applications, bring about a tightening effect of skin, preferably human skin.

### SUMMARY OF THE INVENTION

The invention provides a method of bringing about a tightening effect of skin (preferably human skin) comprising applying to the skin a composition comprising:
an effective amount of a soy protein selected from the group consisting of soy proteins modified by treatment with a reducing agent and subsequently reacted with a carboxylic acid anhydride; soy proteins modified by treatment with a reducing agent, reacted with a carboxylic acid anhydride and subsequently oxidized; soy proteins modified by reaction with a hydroxy alkyl acrylate; soy proteins modified with epoxide, acrylate, or chlorohydrin ionic monomers; soy proteins modified by reaction with an alkyl acrylimidoglycolate; and mixtures thereof.

The composition may also contain at least one cosmetic adjuvant selected from the group consisting of fillers, surfactants, thixotropic agents, antioxidants, preserving agents, dyes, pigments, fragrances, thickeners, vitamins, hormones, moisturizers, UV absorbing organic sunscreens, UV scattering inorganic sunscreens, wetting agents, cationic polymers, anionic polymers, nonionic polymers, and amphoteric polymers.

The effective amount of the modified soy protein is preferably from about 0.001 to about 90% by weight of the total weight of the composition, about 0.001 to about 60% by weight of the total weight of the composition, or about 0.005 to about 20% by weight of the total weight of the composition.

The composition described above can be an anhydrous composition containing a fatty phase, the fatty phase containing at least one liquid, solid, or semi-solid fatty substance, or in the form of an aqueous solution containing mono or polyhydric alcohols. If the composition is an anhydrous composition, the fatty substance preferably is selected from the group consisting of isododecane, hydrogenated polyisobutene, squalane, isononyl isononanoate, cyclotetra- and - pentadimethicones, phenyltrimethicone, ethylene homopolymers, ethoxylated fats and oils, fluoroalkanes, microcrystalline waxes, ozocerite, beeswax, seracite, shea butter, candelilla wax, arachidyl propionate, fluoropolymers represented by the monomer X₁X₂C=CX₃F, wherein X₁, X₂, and X₃ are independently H or F, and copolymers of ethylene and of at least one monomer represented by the formula CH₂ =CH--R₃, wherein R₃ is an alkyl radical containing from 1 to 30 carbon atoms or an aryl or aralkyl radical.

Alternatively, the composition described above can be in the form of a crème emulsion, a gel, a dry powder, an aerosol, a mousse, an alcohol-in-oil emulsion, an alcohol and water solution, an aqueous solution, or an emulsion solution. Preferably, these compositions further comprise a carbomer, gum, or other thickener in a proportion of from about 0.1 to about 15% by weight relative to the total weight of the composition. Alternatively, at least one of the materials found in the composition is in the form of a powder and the effective amount of the modified soy protein is present along with a pigment or filler. More preferably, the effective amount of the modified soy protein is from about 0.001 to about 60% by weight of the total weight of the composition.

The composition described above can also be in the form of a stable dispersion of water-in-oil or oil-in-water type.

### DETAILED DESCRIPTION OF THE INVENTION

The present invention employs compositions comprising an effective amount of a modified soy protein in a suitable cosmetic carrier. The compositions of the present invention include any composition that may be applied to the skin to provide a tightening effect. These compositions include, but are not limited to, skin care, skin cleansing, make-up, facial lotions; crème moisturizers; body washes; body lotions; foot and hand crèmes; lipsticks; eyeshadow; foundations; shaving crème compositions, including gel types; shaving lotions; crème and lotion depilatories; facial masks made with clay materials; and anti-aging products.

The following definitions are used herein and should be referred to for interpretation of the claims and the specification.

The term "skin tightening" refers to the effect of forming a skin firming and toning film on the skin, resulting in a reduction in the visible signs of aging, including fine lines, wrinkles, and sagging skin.

The term "cosmetic" refers to a preparation primarily intended for external use for purposes of beautification or for the sake of appearance.

The terms "polypeptide" and "protein" are used interchangeably. The term "peptide" is used to describe a subunit of a polypeptide or protein formed by hydrolysis.

The term "substantially unhydrolyzed soy proteins" is used to refer to soy proteins that have no significant reduction in the individual subunit molecular weight of the protein material.

The term "partially hydrolyzed soy protein" is used to describe soy proteins that are hydrolyzed to a limited extent to form peptides with a molecular weight in the range of about 2 kDa to about 350 kDa.

The term "natural protein" is used to describe a protein that is isolated from its native animal or plant source.

The term "recombinant protein" is used to describe a protein that is produced by a non-native organism using genetic engineering techniques. Both natural proteins and recombinant proteins can exist in their native sequences or in sequences modified by genetic engineering.

Soy protein materials that are useful for preparing the modified soy proteins include soy flour, soy concentrate and most preferably soy protein isolate and native isolated soy protein. The soy flour, soy concentrate, soy protein isolate, and native isolated soy protein are formed from a soybean starting material, which may be soybeans or a soybean derivative. The soybean starting material may be soybean cake, soybean chips, soybean meal or preferably, soybean flakes. These soybean starting materials may be prepared using conventional procedures known in the art. The soy protein isolate is preferably prepared from defatted soy flake material, as described by Krinski et al. in U.S. Patent No. 5,766,331 (in particular, column 4, lines 41 to 60). The soy protein material is then chemically modified to form the modified soy proteins of the instant invention.

The modified soy proteins include, but are not limited to soy proteins modified by treatment with a reducing agent and subsequently reacted with a carboxylic acid anhydride; soy proteins modified by treatment with a reducing agent, reacted with a carboxylic acid anhydride and subsequently oxidized; soy proteins modified by reaction with a hydroxy alkyl acrylate; soy proteins modified with ionic monomers such as epoxide, acrylate, and chlorohydrin; and soy proteins modified by reaction with an alkyl acrylimidoglycolate. The soy proteins may be used in a substantially unhydrolyzed condition or may be partially hydrolyzed. As used herein, a substantially unhydrolyzed soy protein refers to a soy protein that has no significant reduction in the individual subunit molecular weight of the protein material. Substantially unhydrolyzed soy proteins have a molecular weight from about 10 kDa to about 600 kDa. It is preferred that a substantially unhydrolyzed soy protein be used in the compositions of the instant invention. The larger protein molecules make better surface-modifying agents than the extensively hydrolyzed soy proteins described in the prior art. However, if desired, partially hydrolyzed soy proteins may be used. Methods useful for the partial hydrolysis of soy proteins are well known in the art (see for example Yoshioka et al., U.S. Patent No. 5,753,214), and include, but are not limited, to treatment with acid or base, or with a proteolytic enzyme such as pepsin, papain, trypsin, chymotrypsin, subtilisin, and the like. The time of reaction and the concentration of the reagents may be adjusted to obtain the degree of hydrolysis desired. The partially hydrolyzed soy proteins have a molecular weight of about 2 kDa to about 350 kDa.

The soy proteins modified by treatment with a reducing agent and subsequently reacted with a carboxylic acid anhydride may be prepared using the method described by Coco et al. in U.S. Patent No. 4,474,694 (in particular, column 3, line 57 to column 6, line 15). In that method, an isolated soy protein material is initially prepared by treatment of defatted soybean flakes with an alkaline solution to solubilize the protein. This alkaline treatment step produces three important modifications in the proteins, including 1) hydrolysis of the amides of asparagine and glutamine to carboxylic acids, 2) protein cross-linking through beta elimination of serine and cysteine, followed by reaction with lysine, and 3) protein reorganization by hydrophilic and hydrophobic groups. The protein cross-linking results in the formation of some very high molecular weight fragments, such that the molecular weight range of the modified proteins is from about 2 kDa to about 5,000 kDa. The protein extract is then separated from the alkali-insoluble solids by filtration or centrifugation. This protein extract or dispersion is used as the starting material in the process. This protein extract or dispersion is first treated with a reducing agent such as thioglycolic acid or salts of thioglycolic acid to react with the disulfide bonds and improve the reactivity of the protein material during subsequent reaction. This treated soy protein dispersion may be kept in substantially unhydrolyzed form or may be partially hydrolyzed as described *supra.* The treated soy protein dispersion is then reacted with a carboxylic acid anhydride to modify the protein material and impart unique film-forming properties for the protein material which are useful in compositions, and makeup. The preferred carboxylic acid anhydride is a dicarboxylic acid anhydride such as phthalic anhydride or succinic anhydride. Following the reaction of the soy protein material with the carboxylic acid anhydride, the modified soy protein may be isolated by acid precipitation at the isoelectric point and recovered. These types of modified soy proteins are commercially available, for example from E.I. du Pont de Nemours and Co., Inc. (Wilmington, DE).

The soy proteins modified by treatment with a reducing agent, reacted with a carboxylic acid anhydride and subsequently oxidized may be prepared by the method described by Krinski et al. in U.S. Patent No. 4,961,788 (in particular, column 2, line 58 to column 3, line 12). In that method, soy proteins modified by treatment with a reducing agent and subsequently reacted with a carboxylic acid anhydride are prepared according the method described by Coco et al, *supra.* This modified soy protein is then further modified by oxidation using a bleaching agent, such as hydrogen peroxide. These types of modified soy proteins are commercially available, for example from E.I. du Pont de Nemours and Co., Inc. (Wilmington, DE).

The soy proteins modified by reaction with a hydroxy alkyl acrylate may be prepared by the method described by Steinmetz et al. in U.S. Patent No. 4,687,826 (in particular, column 3, line 8 to column 4, line 41). In that method, an isolated soy protein material is initially prepared by treatment of defatted soybean flakes with an alkaline solution to solubilize the protein. The protein extract is then separated from the alkali-insoluble solids by filtration or centrifugation. The alkaline dispersion of the soy protein may be maintained in substantially unhydrolyzed form or may be partially hydrolyzed, as described *supra.* The soy protein dispersion is then reacted with a hydroxyl alkyl acrylate in an amount sufficient to modify the protein. The preferred hydoxy alkyl acrylate is one in which the alkyl group is C₁ to C₈, and most preferably, C₂ or C₃. The modified soy protein may be isolated by acid precipitation at the isoelectric point and recovered.

The soy proteins modified with ionic monomers such as epoxide, acrylate, and chlorohydrin may be prepared using the method described by Krinski et al. in U.S. Patent No. 4,689,381 (in particular, column 3, line 9 to column 4, line 53). In that method, an isolated soy protein material is initially prepared by treatment of defatted soybean flakes with an alkaline solution to solubilize the protein. The protein extract is then separated from the alkali-insoluble solids by filtration or centrifugation. The alkaline dispersion of the soy protein may be maintained in substantially unhydrolyzed form or may be partially hydrolyzed, as described *supra.* The soy protein dispersion is then reacted with a cationic epoxide monomer, such as 2,3 epoxypropyltrimethyl ammonium chloride, a cationic acrylic monomer, such as methacrylamidopropyltrimethyl ammonium chloride, or a cationic chlorohydrin monomer, such as 3-chloro, 2-hydroxypropyltrimethyl ammonium chloride, in an amount sufficient to modify the protein material. The modified soy protein may be isolated by acid precipitation at the isoelectric point and recovered. These types of modified soy proteins are commercially available, for example from E.I. du Pont de Nemours and Co., Inc. (Wilmington, DE).

The soy proteins modified by reaction with an alkyl acrylimidoglycolate may be prepared by the method described by Krinski et al. in U.S. Patent No. 4,554,337 (in particular, column 3, line 7 to column 4, line 31). In that method, an isolated soy protein material is initially prepared by treatment of defatted soybean flakes with an alkaline solution to solubilize the protein. The protein extract is then separated from the alkali-insoluble solids by filtration or centrifugation. The soy protein dispersion is then reacted with an alkyl acrylamidoglycolate alkyl ether, such as methyl acrylamidoglycolate methyl ether, in an amount sufficient to modify the protein material. The modified soy protein may be isolated by acid precipitation at the isoelectric point and recovered.

In the compositions, the expression "effective amount" of a modified soy protein corresponds to a proportion of from about 0.001 to about 90% by weight, but preferably from about 0.001 to about 60% by weight, most preferably from about 0.005 to about 20% by weight, relative to the total weight of the composition. This proportion may vary as a function of the type of composition.

The compositions may also contain one or more conventional cosmetic or dermatological additives or adjuvants, including, but not limited to, fillers, surfactants, thixotropic agents, antioxidants, preserving agents, dyes, pigments, fragrances, thickeners, vitamins, hormones, moisturizers, UV absorbing organic sunscreens, UV scattering inorganic sunscreens, wetting agents, cationic, anionic, and nonionic or amphoteric polymers. These adjuvants are well known in the field of cosmetics and are described in many publications, for example see Harry's Book of Cosmeticology, 8th edition, Martin Rieger, ed., Chemical Publishing, New York (2000).

Among these adjuvants, the fillers are generally present in products in a maximum proportion of about 99.9% by weight relative to the total weight of the composition. These fillers, in the form of very fine powders, can be of natural or synthetic origin and include, but are not limited to, mineral powders, such as talc, kaolin, mica, silica, silicates, alumina, zeolites, hydroxyapatite, sericite, titanium dioxide, titanium micas, barium sulfate, calcium carbonate, calcium sulfate, bismuth oxychloride, boron nitride and metal powders such as aluminum powder; plant powder, such as corn starch, wheat starch or rice starch powders; organic powders, such as polyamide powder, polyester powder, polytetrafluoroethylene powder, the powder of fluorinated alkanes, polyethylene powder and other inert plastics. These various powders can also be coated, for example with metal salts of fatty acids, amino acids, lecithin, collagen, silicone compounds, fluoro compounds or with any common coating agent.

The compositions may also contain surfactants or wetting agents, preferably at about 0.001 to about 18%, more preferably at about 0.005 to about 15% by weight of the total composition. The terms "surfactants" and "wetting agents" as used herein refer to surface-active agents which, when added to water, cause it to penetrate more,easily into, or spread on the surface of another material, by reducing the surface tension of the water at the water-air or water-oil interface. By "surface active agent" is meant any compound that reduces surface tension when dissolved in water or water solutions. The selection of a surfactant for this purpose presents a wide range of possibilities known in the art. Suitable surfactants include, but are not limited to, the following:
(1) anionic surfactants, such as metallic or alkanolamine salts of fatty acids for example sodium laurate and triethanolamine oleate; alkyl benzene sulfones, for example triethanolamine dodecyl benzene sulfonate; alkyl sulfates, for example sodium lauryl sulfate; alkyl ether sulfates, for example sodium laurel ether sulfate (2 to 8 EO); sulfosuccinates, for example sodium dioctyl sulfonsuccinate; monoglyceride sulfates, for example sodium glyceryl monostearate monosulfate; isothionates, for example sodium isothionate; methyl taurides, for example Igepon T; acylsarcosinates, for example sodium myristyl sarcosinate; acyl peptides, for example Maypons and lamepons;
   acyl lactylates, polyalkoxylated ether glycollates, for example trideceth-7 carboxylic acid; phosphates, for example sodium dilauryl phosphate.
(2) cationic surfactants, such as amine salts, for example sapamin hydrochloride; quaternary ammonium salts, for example Quaternium 5, Quaternium 31 and Quaternium 18;
(3) amphoteric surfactants, such as imidazol compounds, for example Miranol; N-alkyl amino acids, such as sodium
   cocaminopropionate and asparagine derivatives; betaines, for example cocamidopropylebetaine;
(4) nonionic surfactants, such as fatty acid alkanolamides, for example oleic ethanolamide; esters or polyalcohols, for example Span; polyglycerol esters, for example that esterified with C.sub.1 2.sub.-1 8 fatty acids and one or several OH groups; Polyalkoxylated derivatives, for example polyoxy:polyoxyethylene stearate (available for example from McIntyre Co); ethers, for example polyoxyethe lauryl ether (available for example from Stepan Co., Northfield, IL, as Stepanol ES); ester ethers, for example Tween; amine oxides, for example coconut and dodecyl dimethyl amine oxides. Mixtures of two or more of the above surfactants can be employed in the compositions according to the invention.

The compositions may also contain thixotropic or gelling agents, preferably at about 0.02 to about 20%, more preferably at about 0.05 to about 18% by weight of the total composition. Suitable thixotropic or gelling agents include, but are not limited to, stearates of aluminum, calcium, magnesium, potassium, sodium, or zinc; hydroxystearate, isostearate, laurate, linoleate, myristate, oleate, olivate, palmate, palmitate, tallowate, rosinate, and the like, and fatty acid esters of glycol, triglycerides, mixtures of fatty alcohols, cholesterol derivatives and in particular hydroxycholesterol, and clay minerals which swell in the presence of oil, and in particular those belonging to the montmorillonite group.

The compositions may also contain antioxidants, preferably at about 0.001 to about 10%, more preferably at about 0.01 to about 8% by weight of the total composition. Suitable antioxidants are ingredients which assist in preventing or retarding spoilage. Examples of antioxidants suitable for use in the compositions of the invention include, but are not limited to, potassium sulfite, sodium bisulfite, sodium erythrobate, sodium metabisulfite, sodium sulfite, propyl gallate, cysteine hydrochloride, butylated hydroxytoluene, butylated hydroxyanisole, and the like.

The compositions may also contain preserving agents, preferably at about 0.001 to about 8%, more preferably at about 0.01 to about 5% by weight of the total composition. Suitable preserving agents include, but are not limited to, benzoic acid, benzyl alcohol, benzylhemiformal, benzylparaben, 5-bromo-5-nitro-1,3- dioxane, 2-bromo-2-nitropropane-1,3-diol, butyl paraben, phenoxyethanol, methyl paraben, ethyl paraben, propyl paraben, diazolidinyl urea, calcium benzoate, calcium propionate, captan, chlorhexidine diacetate, chlorhexidine digluconate, chlorhexidine dihydrochloride, chloroacetarnide, chlorobutanol, p-chloro-m-cresol, chlorophene, chlorothymol, chloroxylenol, m-cresol, o-cresol, DEDM Hydantoin, DEDM Hydantoin dilaurate, dehydroacetic acid, diazolidinyl urea, dibromopropamidine diisethionate, DMDM Hydantoin, Phenonip^{®}, Kathon^{®} and all of those disclosed on pages 570 to 571 of the CTFA Cosmetic Ingredient Handbook, Second Edition, 1992, which is herein incorporated by reference.

The compositions may also contain dyes, preferably at about 0.1 to about 15%, by weight of the total composition. Suitable dyes include, but are not limited to, eosin derivatives such as D&C Red No. 21 and halogenated fluorescein derivatives such as D&C Red No. 27, D&C Red Orange No. 5 in combination with D&C Red No. 21 and D&C Orange No. 10.

The compositions may also contain pigments, preferably at about 0.1 to about 15% by weight of the total composition. Suitable pigments may be inorganic or organic or alternatively metal lakes and include, but are not limited to, titanium dioxide, zinc oxide, barium oxide, D&C Red No. 36 and D&C Orange No. 17, the calcium lakes of D&C Red Nos. 7, 11, 31 and 34, the barium lake of D&C Red No. 12, the strontium lake D&C Red No. 13, the aluminum lakes of FD&C Yellow No. 5, of FD&C Yellow No. 6, of D&C Red No. 27, of D&C Red No. 21, and of FD&C Blue No. 1, iron oxides, manganese violet, chromium oxide, ultramarine blue, and carbon black particles.

The compositions may also contain fragrances, preferably at about 0.01 to about 10%, by weight of the total composition. Numerous fragrances, both natural and synthetic, are well known in the art. For example, Secondini (Handbook of Perfumes and Flavors, Chemical Publishing Co., Inc., New York, 1990), incorporated herein by reference, describes many of the natural and synthetic fragrances used in cosmetics. Suitable natural fragrances include, but are not limited, to jasmines, narcissus, rose, violet, lavender, mint, spice, vanilla, anise, amber, orange, pine, lemon, wintergreen, rosemary, basil, and spruce. Suitable synthetic fragrances include, but are no limited to, acetaldehyde, C7 to C16 alcohols, benzyl acetate, butyric acid, citric acid, isobutyl phenyl acetate, linalyl butyrate, malic acid, menthol, phenyl ethyl cinnamate, phenyl propyl formate, tannic acid, terpineol, vanillin, amyl salicylate, benzaldehyde, diphenyl ketone, indole, and the like.

The compositions may also contain thickeners, preferably at about 0.001 to about 25%, more preferably at about 0.1 to about 15%, by weight of the total composition. Suitable thickeners include, but are not limited to, starch; gums, such as gum arabic or xanthan gum; carbomer polymers, such as Carbopol^{®} 941, 940, 934 (available from Union Carbide Co., Midland, MI), and Ultrez 10; kaolin or other clays, ethylene glycol monostearate, carboxyvinyl polymer, acrylic copolymers, hydroxyethyl cellulose, and hydroxypropyl cellulose.

The compositions may also contain vitamins and/or coenzymes, preferably at about 0.001 to about 10%, more preferably at about 0.01 % to about 8%, most preferably at about 0.05% to about 5% by weight of the total composition. Suitable vitamins include, but are not limited to, ascorbic acid and derivatives thereof; the B vitamins, such as thiamine, riboflavin, pyridoxin, and the like; vitamin A and derivatives thereof; vitamin E and derivatives thereof; vitamin D and vitamin K; as well as coenzymes such as thiamine pyrophosphate, flavin adenine dinucleotide, folic acid, pyridoxal phosphate, tetrahydrofolic acid, and the like.

The compositions may also contain hormones, preferably at about 0.0001 to about 0.01 % by weight of the total composition. Suitable hormones include, but are not limited to, estrogen, progesterone, pregnenolone, testosterone, estradiol, hydrocortisone, and cortisone.

The compositions may also contain moisturizers, preferably at about 0.1 to about 30%, more preferably at about 0.5 to about 25%, most preferably at about 1 to about 20% by weight of the total composition. These moisturizers include water-soluble, low molecular weight moisturizers, fat-soluble, low molecular weight moisturizers, water-soluble, high molecular weight moisturizers and fat-soluble, high molecular weight moisturizers. Suitable water-soluble, low molecular weight moisturizers include, but are not limited to, serine, glutamine, sorbitol, mannitol, pyrrolidone-sodium carboxylate, glycerin, propylene glycol, 1,3-butylene glycol, ethylene glycol, polyethylene glycol (polymerization degree n=2 or more), polypropylene glycol (polymerization degree n=2 or more), polyglycerin (polymerization degree n=2 or more), lactic acid and lactate. Suitable fat-soluble, low molecular weight moisturizers include, but are not limited to, cholesterol and cholesterol ester. Suitable water-soluble, high molecular weight moisturizers include, but are not limited to, carboxyvinyl polymers, polyaspartate, tragacanth, xanthane gum, methyl cellulose, hydroxymethyl cellulose, hydroxyethyl cellulose, hydroxypropyl cellulose, carboxymethyl cellulose, water-soluble chitin, chitosan and dextrin. Suitable fat-soluble, high molecular weight moisturizers include, but are not limited to, polyvinylpyrrolidone-eicosene copolymers, polyvinylpyrrolidone-hexadecene copolymers, nitrocellulose, dextrin fatty acid ester and high molecular silicone.

The compositions may also contain UV absorbing organic sunscreens, preferably at about 0.001 to about 20%, more preferably at about 0.01 to about 10%, most preferably at about 0.05 to about 8% by weight of the total composition. UV absorbing organic sunscreens are herein defined as organic chemicals that absorb ultraviolet light of wavelengths between 290 and 329 nm. Suitable UV absorbing organic sunscreens include, but are not limited to, para-aminobenzoic acid, ethyl para-aminobenzoate, amyl para-aminobenzoate, octyl para-aminobenzoate, ethylene glycol salicylate, phenyl salicylate, octyl salicylate, benzyl salicylate, butylphenyl salicylate, homomenthyl salicylate, benzyl cinnamate, 2-ethoxyethyl para-methoxycinnamate (such as Parsol^{®} available from Givaudan-Roure Co.), octyl para-methoxycinnamate, glyceryl mono(2-ethylhexanoate) dipara-methoxycinnamate, isopropyl para-methoxycinnamate, diisopropyl-diisopropylcinnamic acid ester mixtures, urocanic acid, ethyl urocanate, hydroxymethoxybenzophenone, hydroxymethoxybenzophenonesulfonic acid and salts thereof, dihydroxymethoxybenzophenone, sodium dihydroxymethoxybenzophenonedisulfonate, dihydroxybenzophenone, tetrahydroxybenzophenone, 4-tert -butyl-4'-methoxydibenzoylmethane, 2,4,6- trianilino-p -(carbo-2'-ethylhexyl-1'-oxy)-1,3,5-triazine, and 2-(2-hydroxy-5-methylphenyl)benzotriazole. UV scattering inorganic sunscreen materials, such as inorganic pigments and metal oxides, including but not limited to oxides of titanium (such as SunSmart available from Cognis Co.), zinc, and iron, may also be incorporated into the compositions of the instant invention. UV scattering inorganic sunscreens are herein defined as inorganic substances that scatter ultraviolet light of wavelengths between 210 and 280 nm. These UV scattering inorganic sunscreens may be used in the compositions of this invention at concentrations of preferably about 0.001 to about 40%, more preferably at about 0.01 to about 10%, most preferably at about 0.05 to about 8% by weight of the total composition.

The compositions may also contain other film-forming polymers, preferably at about 0.01 to about 20%, more preferably at about 0.01 % to about 10%, by weight of the total composition. These polymers serve as conditioners to coat the skin, or to coat particles that are present in the composition. These polymers may be cationic, anionic, nonionic, or amphoteric. Cationic polymers are herein defined as synthetic or natural polymers that contain, or have been modified to contain, positively charged groups and/or groups that can ionize to positively charged groups. Suitable cationic polymers, include, but are not limited to, cationized cellulose, cationized guar gum, diallyly quaternary ammonium salt/acrylamide copolymers, quaternized polyvinylpyrrolidone and derivatives thereof, polyquaternium-1, polyquaternium-2, polyquaternium-5, polyquaternium-6, polyquaternium-7, polyquatemium-8, polyquaternium-9, polyquaternium-11, polyquaternium-12, polyquaternium-13, polyquaternium-14, polyquaternium-15, polyquaternium-16, polyquaternium-17, polyquaternium-18, polyquaternium-19, polyquaternium-20, polyquaternium- 22, polyquaternium-27, polyquaternium-28, polyquaternium-29, polyquaternium-30, and mixtures thereof, wherein the compound designation is the name adopted for the compound by the Cosmetic, Toiletry and Fragrance Association, and found in the CTFA International Cosmetic Ingredient Dictionary, J. Nikitakis, ed., Cosmetic, Toiletry and Fragrance Association, Inc., Washington, D.C. (1991).

Anionic polymers are herein defined as synthetic or natural polymers that contain, or have been modified to contain, negatively charged groups and/or groups that can ionize to negatively charged groups. Suitable anionic polymers, include, but are not limited to, polyacrylic acid, polymethacrylic acid, carboxymethylcellulose, hydroxymethylcellulose, and starch.

Nonionic polymers are herein defined as synthetic or natural polymers that do not contain any charged groups. Suitable nonionic polymers, include, but are not limited to, polyvinylpyrrolidone, polyvinyl alcohol, polyethylene glycol, polyvinylacetate, polysiloxanes, and copolymers of vinylpyrrolidone and vinyl acetate.

Amphoteric polymers are herein defined as synthetic or natural polymers that contain both negatively and positively charged groups and/or groups that can ionize to give positively and negatively charged groups. Suitable amphoteric polymers are described by Marchi et al. In U.S. Patent No. 5,643,672. Examples include, but are not limited to, polymers resulting from the copolymerization of a monomer derived from a vinyl compound carrying a carboxyl group, such as acrylic acid, methacrylic acid, maleic acid and alpha-chloroacrylic acid, and a basic monomer derived from a substituted vinyl compound containing at least one basic nitrogen atom, such as dialkylaminoalkyl methacrylates and acrylates and dialkylaminoalkylmethacrylamides and-acrylamides, products sold by the company National Starch under the name Amphomer^{®}, methyl methacrylate/ethyldimethylcarboxymethylammonium methacrylate copolymers, such as the products sold by Chimex under the name Mexomer PX (CTFA name: "polyquaternium-30"), methacryloylethylbetaine/methacrylate copolymer sold by Sandoz under the name Diaformer, the methacryloylethylbetaine/methacrylate copolymer sold by Amerchol under the name Amersette, polysiloxane polyorganobetaine copolymers sold by Goldschmidt under the name Abil B 9950 (CTFA Name: "Dimethicone PropyIPG-Betaine"), the polydimethylsiloxane containing alkylphosphobetaine groups sold by Siltech under the name Pecosil SPB-1240, and the oxyethyleneoxypropylene organobetaine/siloxane copolymer sold by Goldschmidt under the name BC 1610.

The compositions may also include an effective amount of one or more other natural or recombinant proteins that enhance the film-forming and coating properties of the modified soy proteins or provide other desirable properties. An effective amount of these additional proteins is herein defined as a proportion from about 0.001 to about 60% by weight, preferably from about 0.01 to about 20% by weight relative to the total weight of the composition. This proportion may vary as a function of the type of composition. Every protein molecule may be considered to be a polymer of amino acids. There are 20 naturally occurring amino acids, each with a common backbone combined with one of 20 variable side chains. Amino acids are commonly classified as either neutral, anionic or cationic amino acids based on the charge of the variable side chain. The naturally occurring anionic amino acids include aspartic acid and glutamic acid, both of which have a carboxylate group at the termini of their respective variable side chains. The naturally occurring cationic amino acids include lysine, arginine and histidine, all of which comprise an amino group in their respective variable side chains. Amino acids may also be modified to result in a net cationic charge. All such amino acids are considered herein to be "cationic amino acids".

The most advantageous proteins to use in the composition will depend on the specific composition. For example, skin care products are generally in the pH range of 2.0 to 10.0. Proteins having a predominance of anionic amino acids, relative to cationic amino acids, are likely to be negatively charged at the general pH range of these products. Conversely, proteins having a relative abundance of cationic amino acids are likely to be positively charged under similar conditions. It is preferred that the amino acid composition of the additional natural or recombinant proteins of the present invention contain a preponderance of anionic amino acids relative to cationic amino acids, so that the proteins are negatively charged at the general pH range of skin care and products. Specifically, the compositions of the present invention preferably comprise an additional natural or recombinant protein, wherein the molar ratio of anionic amino acids to cationic amino acids is at least 1.1:1, preferably from about 1.1:1 to 25:1, and more preferably from about 1.1:1 to 15:1. The term "molar ratio of anionic amino acids to cationic amino acids" of the natural or recombinant protein in the present invention is herein defined as the total molar amount of anionic amino acids (for example, adding the individual molar amount of the anionic amino acids, such as aspartic acid and glutamic acid) in relation to the total molar amount of cationic amino acids (for example, adding the individual molar amount of lysine, arginine and histidine) in the protein. The molar ratio of anionic amino acids to cationic amino acids may be readily calculated based on the overall amino acid composition of the natural or recombinant protein or mixtures of natural or recombinant proteins. Such compositions are frequently known in the art. Alternatively, the amino acid composition of any protein may be determined using techniques known to those skilled in the art, which include, but are not limited to automated amino acid analysis and high pressure liquid chromatography. Such techniques separate the individual amino acids, and the molar ratio of anionic to cationic amino acids may be determined.

Furthermore, the additional natural or recombinant proteins for use in the compositions may comprise sulfur-containing amino acids to provide a protective effect against ultraviolet light or chemical damage to the skin. The preferred natural or recombinant proteins of this invention are characterized in that they contain at least about 0.25%, preferably from about 0.25 to about 15%, and more preferably from about 0.25 to about 5.0% by weight of a sulfur containing amino acid. The term "sulfur-containing amino acids" is herein defined as any amino acid, natural or synthetic, containing sulfur in any form, including, but not limited to, sulfhydryl groups or disulfide bonds. Sulfur-containing amino acids suitable for the present invention include, but are not limited to, cysteine, cystine, methionine, and their respective derivatives and synthetic analogues.

The preferred natural or recombinant proteins for use in this invention include, but are not limited to, wheat and oat proteins, collagen, keratins, gelatin, elastin, fibronectin, soluble reticulin, and water-soluble silk proteins, prepared as described by Fahnestock in copending U.S. Provisional Patent Application No. 60/425617. The natural or recombinant proteins may be used in their native form or they may be modified to alter their ionic charge, for example by sulfonation or succinylation. These proteins may also be in partially hydrolyzed form by cleaving them into smaller peptide units, as described *supra.* These additional proteins may also include unmodified soy proteins in unhydrolyzed or hydrolyzed form.

According to one embodiment of the present invention, the compositions are anhydrous and comprise a fatty phase in a proportion generally of from about 10 to about 90% by weight relative to the total weight of the composition, wherein the fatty phase contains at least one liquid, solid or semi-solid fatty substance. The fatty substances include, but are not limited to oils, fats, waxes, gums, and so-called pasty fatty substances. The oils in the fatty phase may be of mineral, animal, plant or synthetic origin, and may or may not be volatile at room temperature. Oils of mineral origin include, but are not limited to, liquid paraffin and liquid petroleum jelly. Oils of animal origin include, but are not limited to, squalene and squalane. Oils of plant origin include, but are not limited to, sweet almond oil, beauty-leaf oil, palm oil, avocado oil, jojoba oil, sesame oil, olive oil, castor oil and cereal germ oils such as, for example, wheatgerm oil. Synthetic oils include, but are not limited to:
(1) esters of the following formula: R₁-COOR₂

   in which:

   R₁ represents a higher fatty acid residue containing from 7 to 20 carbon atoms, and
   R₂ represents a hydrocarbon-based radical containing from 3 to 30 carbon atoms.
   These esters, include, but are not limited to: purcellin oil, butyl myristate, isopropyl myristate, cetyl myristate, isopropyl palmitate, butyl stearate, hexadecyl stearate, isopropyl stearate, octyl stearate, isocetyl stearate, decyl oleate, hexyl laurate, isononyl isononanoate and esters derived from lanolic acid, such as isopropyl lanolate and isocetyl lanolate. Other synthetic oils include, but are not limited to, isododecane (available for example from Exxon-Mobil Chemical Co., Houston, TX, under the trade name of ISOBAR , isohexadecane, polyisobutenes and hydrogenated polyisobutene, as well as acetylglycerides, octanoates and decanoates of polyalcohols such as those of glycol and of glycerol, ricinoleates of alcohols or of polyalcohols, such as cetyl ricinoleate, propylene glycol dicaprylate and diisopropyl adipate;
(2) fatty alcohols including, but not limited to, oleyl alcohol, linoleyl alcohol, linolenyl alcohol, isostearyl alcohol and octyldodecanol;
(3) ethoxylated oils and fats, including but not limited to, triglycerides with a polyethylene glycol chain inserted, ethoxylated mono and di-glycerides, polyethoxylated lanolins, ethoxylated butter derivatives, polyethylene glycol derivatives of glyceryl cocoate, glyceryl caproate, glyceryl caprylate, glyceryl tallowate, glyceryl palmate, glyceryl stearate, glyceryl laurate, glyceryl oleate, glyceryl ricinoleate, and glyceryl fatty esters derived from triglycerides, such as palm oil, almond oil, and corn oil, glyceryl tallowate, glyceryl cocoate, and polyethylene glycol based polyethoxylated fatty alcohols such as PEG 40 hydrogenated castor oil (commercially available under the tradename Cremophor (RTM) from BASF), PEG 7 glyceryl cocoate and PEG 20 glyceryl laurate (commercially available from Henkel under the tradenames Cetiol (RTM) HE and Lamacit (RTM) GML 20 respectively), and polyethylene glycol ethers of ceteryl alcohol such as Ceteareth 25 (available from BASF under the trade name Cremaphor A25).
(4) silicone oils including, but not limited to, optionally functionalized linear polydiorganosiloxanes, cyclic polydiorganosiloxanes and in particular cyclotetra- and -pentadimethicones and organopolysiloxanes such as alkyl, alkoxy or phenyl dimethicones, and in particular phenyltrimethicone (available from Dow Corning, Midland, MI, as Simethicone and DC 200 Fluids);
(5) fluoro oils including, but not limited to, fluoroalkanes and fluoropolyethers, partially fluorinated hydrocarbon-based oils, and fluoropolymers represented by the monomer unit:

   X₁X₂C=CX₃F

   wherein X₁, X₂, and X₃ are independently H or F.

The waxes in the fatty phase may be of mineral, fossil, animal, plant or synthetic origin or alternatively can be hydrogenated oils or fatty esters which are solid at 25 °C. The mineral waxes, include, but are not limited to, microcrystalline waxes, paraffin, petroleum jelly and ceresine. The fossil waxes, include, but are not limited to, ozocerite and montan wax. The waxes of animal origin, include, but are not limited to beeswax, spermaceti, lanolin wax and derivatives obtained from lanolin such as lanolin alcohols, hydrogenated lanolin, hydroxylated lanolin, acetylated lanolin, lanolin fatty acids and acetylated lanolin alcohol. The waxes of plant origin, include, but are not limited to, candelilla wax, carnauba wax, Japan wax and cocoa butter. The synthetic waxes, include, but are not limited to, ethylene homopolymers, seracite, shea butter, and copolymers of ethylene and of a monomer corresponding to the formula:

CH₂ =CH--R₃

in which: R₃ represents an alkyl radical containing from 1 to 30 carbon atoms or an aryl or aralkyl radical. The alkyl radical of 1 to 30 carbon atoms is preferably a methyl, ethyl, propyl, isopropyl, butyl, decyl, dodecyl or octadecyl radical. Waxes obtained by Fisher-Tropsch synthesis and silicone waxes may also be used.

The hydrogenated oils which are solid at 25 °C include, but are not limited to, hydrogenated castor oil, hydrogenated palm oil, hydrogenated tallow and hydrogenated coconut oil. The fatty esters which are solid at 25 °C include, but are not limited to, propylene glycol monomyristate and myristyl myristate. Waxes which can be used in the compositions according to the invention include, but are not limited to, cetyl alcohol, stearyl alcohol, mono-, di- and triglycerides which are solid at 25 °C, stearic monoethanolamide, colophony and its derivatives such as glycol abietate and glyceryl abietate, sucroglycerides and calcium, magnesium, zinc and aluminum oleates, myristates, lanolates, stearates and dihydroxystearates.

The pasty-type fatty substances can be of mineral, animal, plant or synthetic origin. The pasty fatty substances include, but are not limited to, synthetic esters such as arachidyl propionate, polyvinyl laurate, polyethylene waxes and organopolysiloxanes such as alkyldimethicones, alkoxydimethicones or dimethicone esters.

These anhydrous compositions can be in various forms including, but not limited to, an oily gel, solid products, such as compacted or cast powders, or alternatively sticks such as, for example lipsticks. When the compositions according to the present invention are in the form of an oily gel, they generally contain a thixotropic or gelling agent, examples of which are given *supra.* The thixotropic agents can be present in various proportions depending on the desired texture of the compositions. However, in most cases, they are present in a proportion of from about 1 to about 20% by weight relative to the total weight of the composition.

The anhydrous compositions may be used in particular as skin care, skin cleansing, or make-up products. When they are present in the form of make-up products, they can be foundations, mascaras, eyeliners, lipsticks, eyeshadows or blushers. These compositions are generally colored and contain dyes and/or pigments as cosmetic adjuvants, which are described *supra.*

According to a another embodiment of the present invention, the compositions are stable dispersions in the form of a water-in-oil (W/O) or oil-in-water (O/W) emulsion, which comprise: a fatty phase, as described *supra,* in a proportion of from about 0.1 to about 50% by weight relative to the total weight of the emulsion, the said fatty phase containing at least one modified soy protein, in a proportion of from about 0.001 to about 60% by weight relative to the total weight of the emulsion; an aqueous phase in a proportion of from about 50 to about 98.9% by weight relative to the total weight of the emulsion; and at least one emulsifier in a proportion of from about 1 to about 10% by weight relative to the total weight of the emulsion. Suitable emulsifiers are well known in the field of cosmetic products. For example, water-in-oil emulsifiers include, but are not limited to, sterols such as cholesterol and its associated esters and alcohols, lanolin, calcium oleate and other fatty acid soaps of divalent metals, beeswax, and polyhydric alcoholics of fatty acids such as glyceryl monostearate and sorbitan sesquioleate. Suitable oil-in-water emulsifiers include, but are not limited to, ordinary soaps, partially sulfated fatty alcohols, Cetomacrogol B.P., polyethoxylated esters known as Spans, cetydimethylbenzyl ammonium chloride, and gums and gum substitutes

These emulsions, which are in the form of creams, have good film-forming properties and give a very satisfactory sensation after they have been applied. Such emulsions can be used as skin care, skin cleansing, or make-up products. When these compositions are skin care products, they can be anti-wrinkle products for improving the appearance of the skin. When these compositions are make-up products, they may be foundations or mascaras, containing a certain proportion of the pigments and/or dyes described *supra.*

In order to bring about a skin tightening effect of skin, the compositions may be applied to the skin by various means, including, but not limited to, spraying, brushing, and applying by hand.

### EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the scope thereof, can make various changes and modifications of the invention to adapt it to various uses and conditions.

All ingredients used in the preparation of the compositions described in the following Examples are available commercially unless otherwise noted.

### Example 1

### Tightening effect ex-vivo

### Principle

The principle of Example 1 was the measurement of skin displacement in response to a small sinusoidal force applied parallel to the surface of the skin. The determined parameter is the dynamic spring rate (DSR) (force divided by displacement). The higher the displacement in relation to the force applied, the more supple is the skin and vice versa. Skin softening results in a decrease of DSR, whereas skin tightening results in an increase in DSR.

### Method

A sample of human skin (treated with the substance to be tested) was mounted on a microscope slide. The sample was equilibrated for 2 hours in an atmosphere with controlled humidity (relative humidity, RH = 33%) and temperature (t = 20 °C). After that, the mechanical properties were tested (180 min after the treatment with the substance to be tested). Four samples were tested:
a) control; without treatment
b) skin treated with a placebo formulation (containing polyacrylamide and paraffin)
c) skin treated with the polymer SY1912 (unmodified soy protein isolate 12 % by weight in water) at a concentration of 3.85 % by weight in the placebo formulation
d) skin treated with the polymer SY2515 (15 % by weight in water of SY2500; SY2500 is a soy protein modified by treatment with a reducing agent, reacted with phthalic acid anhydride and subsequently oxidized, which is available from E.I. du Pont de Nemours and Co., Wilmington, DE) at a concentration of 4.55 % by weight in the placebo formulation The amount of polymer applied to the skin was the same in test c) and d): 4 mg/cm²_{.}

The results of six identical assays, expressed as the increase of DSR parameter after 180 min, are summarized in Tables 1-3.

**Table 1**

| Skin Tightening Effect of Unmodified Soy Protein | | | |
|---|---|---|---|
| DSR, 180 minutes after treatment | Control | Placebo | SY1912 |
| Assay 1 | 97.59% | 98.72% | 102.19% |
| Assay 2 | 103.17% | 101.48% | 109.73% |
| Assay 3 | 98.70% | 104.17% | 174.94% |
| Assay 4 | 95.72% | 110.09% | 121.51% |
| Assay 5 | 103.03% | 105.58% | 114.81% |
| Assay 6 | 100.92% | 104.84% | 139.28% |
| average | 99.86% | 104.15% | 127.07% |
| SEM (average of the standard deviation) | 1.24% | 1.57% | 10.87% |
| Statistics | | NS/Control | S/Control S/Placebo |

"NS/Control", "S/Control" and "S/Placebo" are statistical terms. NS/Control means not significant referring to the control without treatment; S/Control means significant relative to the control without treatment and S/Placebo means significant relative to the placebo formulation.

**Table 2**

| Skin Tightening Effect of Modified Soy Protein | | | |
|---|---|---|---|
| DSR, 180 minutes after treatment | Control | Placebo | SY2515 |
| Assay 1 | 94.96% | 85.90% | 133.94% |
| Assay 2 | 102.81% | 115.36% | 157.39% |
| Assay 3 | 98.70% | 104.17% | 202.22% |
| Assay 4 | 99.65% | 110.93% | 182.65% |
| Assay 5 | 106.90% | 99.79% | 149.45% |
| Assay 6 | 100.92% | 104.84% | 194.23% |
| Average | 100.66% | 103.50% | 169.98% |
| SEM (average of the standard deviation) | 1.64% | 4.17% | 11.06% |
| Statistics | | NS/Control | S/Control S/Placebo |

**Table 3**

| Comparison of the Skin Tightening Effect Obtained with Unmodified and Modified Soy Protein | | |
|---|---|---|
| % increase of DSR relative to placebo (%) | SY1912 | SY2515 |
| Assay 1 | 3.51% | 55.92% |
| Assay 2 | 8.12% | 36.43% |
| Assay 3 | 67.94% | 94.13% |
| Assay 4 | 10.36% | 64.66% |
| Assay 5 | 8.74% | 49.77% |
| Assay 6 | 32.85% | 85.26% |
| Average | 21.92% | 64.36% |
| SEM | 10.12% | 8.92% |
| Statistics | | S/SY1912 |

S/SY1912 means that the results for the modified soy polymer SY2515 are significant relative to the other, unmodified soy polymer SY1912.

Statistical analysis of results: ANOVA test a posteriori PLSD Fisher S = significant (p < 0.05) (The results are significant if p < 0.05)

### Conclusion

The polymers SY1912 and SY2515 showed good tightening activity on human skin ex-vivo. For both products the increase in the DSR value 180 min after application is significant relative to the placebo sample. The chemical modification of natural soy protein improved the desired tightening activity. The difference between the two polymers SY1912 and SY2515 is significant. SY2515 shows a greater tightening effect than SY1912.

### Example 2

### Tightening effect in-vivo

### Principle

The tightening effect in vivo was evaluated using a corneospinometer (as disclosed in French patent FR-B 2 822 672). This device allows the evaluation of the behavior of the Stratum Corneum under microtorsion. The angular deformation of skin was measured in relation to a mechanical torque applied to the skin surface by a probe, which comprises a small needle (0.8 millimeters in diameter). The contact area was below 0.2 square millimeters and the measurement was limited to the Stratum Corneum layer. A special technology allows the lowering of the needle to the skin in the axis of the probe, so that only the weight of the needle is applied to the skin and parasitic friction is decreased. The device comprises a coil which has two functions. Firstly, it applies a periodic torque to the needle. Secondly, it measures the angular positioning of the needle.

The result is expressed by a Dynamic Spring Rate (DSR), which is the ratio between the mechanical torque (constant sinusoidal amplitude) and the angular deformation of the skin (variable). An increase of DSR corresponds to a tightening effect on the Stratum Corneum.

### Products tested

SY 2500, powder form (SY2500 is a soy protein modified by treatment with a reducing agent, reacted with phthalic acid anhydride and subsequently oxidized, which is available from E.I. du Pont de Nemours and Co., Wilmington, DE).

### Method/Test conditions (in key words)

- 1 volunteer
- forearm internal face
- area : 3 x 3 cm²
- applied dose was 2 mg/cm²
- mean value of 50 measurements
- results are expressed as % of increase of DSR after 10 minutes after treatment referring to To (0 minutes after treatment)

The skin was treated with the following composition (weight %):

| | |
|---|---|
| SY 2500 | 2.00% |
| Elestab^{®} 388 | 2.50% |
| Keltrol^{®} T | 0.20% |
| NaOH (10 % in water) | 0.50% |
| Carbopol^{®} 980 2% | 10.00% |
| water | remaining difference to 100% |

The INCl name of Elestab^{®} 388 is "propylene glycol and phenoxethanol and chlorphenesin and methylparaben".
The INCl name of Keltrol^{®} T is "xanthan gum".
The INCl name of Carbopol^{®} 980 is "Carbomer".

The results are given in Table 4.

**Table 4**

| Skin Tightening Effect Obtained with Modified Soy Protein in In-Vivo Testing | |
|---|---|
| | % increase of DSR (T₁₀/T₀) |
| SY 2500 (powder form) at a concentration of 2% by weight | 93.3 |

### Conclusion:

The modified soy protein, SY 2500 (powder form) exhibited a very good immediate tightening effect as demonstrated by the increase of DSR at 10 minutes (T₁₀) compared to T₀.

## Claims

1. A method of bringing about a tightening effect of skin (preferably human skin) comprising applying to the skin a composition comprising an effective amount of a soy protein selected from the group consisting of soy proteins modified by treatment with a reducing agent and subsequently reacted with a carboxylic acid anhydride; soy proteins modified by treatment with a reducing agent, reacted with a carboxylic acid anhydride and subsequently oxidized; soy proteins modified by reaction with a hydroxy alkyl acrylate; soy proteins modified with epoxide, acrylate, or chlorohydrin ionic monomers; soy proteins modified by reaction with an alkyl acrylimidoglycolate; and mixtures thereof.

2. The method of Claim 1 wherein said composition further comprises at least one cosmetic adjuvant selected from the group consisting of fillers, surfactants, thixotropic agents, antioxidants, preserving agents, dyes, pigments, fragrances, thickeners, vitamins, hormones, moisturizers, UV absorbing organic sunscreens, UV scattering inorganic sunscreens, wetting agents, cationic, anionic, and nonionic or amphoteric polymers.

3. The method of Claim 1 wherein the effective amount of the soy protein is from 0.001 to 90% by weight of the total weight of the composition.

4. The method of Claim 1 wherein the effective amount of the soy protein is from 0.001 to 60% by weight of the total weight of the composition.

5. The method of Claim 1 wherein the effective amount of the soy protein is from 0.005 to 20% by weight of the total weight of the composition.

6. The method of Claim 1 wherein the composition further comprises an effective amount of a natural or recombinant protein, or a hydrolyzed natural or recombinant protein.

7. The method of Claim 6 wherein the effective amount of the natural or recombinant protein is from 0.001 to 60% by weight of the total weight of the composition.

8. The method of Claim 6 wherein the effective amount of the natural or recombinant protein is from 0.01 to 20% by weight of the total weight of the composition.

9. The method of Claim 6 wherein the natural or recombinant protein is selected from the group consisting of wheat, oat, and rice proteins, collagen, keratins, gelatin, elastin, fibronectin, soluble reticulin, unmodified soy proteins in unhydrolyzed or hydrolyzed form, and water-soluble silk proteins.

10. The method of Claim 1, wherein the composition is an anhydrous composition and contains 10 to 90% by weight, relative to the total weight of the composition, of a fatty phase, wherein the fatty phase contains at least one liquid, solid or semi-solid fatty substance.

11. The method of Claim 10, wherein the fatty substance is selected from the group consisting of isododecane, hydrogenated polyisobutene, squalane, isononyl isononanoate, cyclotetra- and - pentadimethicones, phenyltrimethicone, ethylene homopolymers, ethoxylated fats and oils, fluoroalkanes, microcrystalline waxes, ozocerite, beeswax, seracite, shea butter, candelilla wax, arachidyl propionate, fluoropolymers represented by the monomer:
X₁X₂C=CX₃F
wherein X₁, X₂, and X₃ are independently H or F, and copolymers of ethylene and of at least one monomer represented by the formula:
CH₂ =CH-R₃
wherein R₃ is an alkyl radical containing from 1 to 30 carbon atoms or an aryl or aralkyl radical.

12. The method of Claim 1, wherein the composition is in the form of an aqueous solution containing mono or polyhydric alcohols.

13. The method of Claim 1, wherein the composition is in the form of a crème emulsion, a gel, a dry powder, an aerosol; a mousse, an alcohol-in-oil emulsion, an alcohol and water solution, an aqueous solution, or an emulsion solution.

14. The method of Claim 13 wherein the composition further comprises a carbomer, gum, or other thickener in a proportion of from 0.1 to 15% by weight relative to the total weight of the composition.

15. The method of Claim 13 wherein at least one of the materials is in the form of a powder and wherein the effective amount of the soy protein is present along with a pigment or filler.

16. The method of Claim 15 wherein the effective amount of the soy protein is from 0.001 to 60% by weight of the total weight of the composition.

17. The method of Claim 15 wherein the pigment is selected from the group consisting of titanium dioxide, zinc oxide, barium oxide, D&C Red No. 36 and D&C Orange No. 17, the calcium lakes of D&C Red Nos. 7, 11, 31 and 34, the barium lake of D&C Red No. 12, the strontium lake D&C Red No. 13, the aluminum lakes of FD&C Yellow No. 5, of FD&C Yellow No. 6, of D&C Red No. 27, of D&C Red No. 21, and of FD&C Blue No. 1, iron oxides, manganese violet, chromium oxide, ultramarine blue, and carbon black particles.

18. The method of Claim 15 wherein the filler is selected from the group consisting of calcium carbonate, aluminum silicate, calcium silicate, magnesium silicate, mica, talc, barium sulfate, calcium sulfate, powdered polyamides, powdered polyesters, powdered fluorinated alkanes, and other inert powdered plastics.

19. The method of Claim 1, wherein the composition is in the form of a stable dispersion of water-in-oil or oil-in-water type, and comprises:
a) a fatty phase in a proportion of from 0.1 to 50% by weight relative to the total weight of the composition, wherein the fatty phase contains the soy protein in a proportion of from 0.001 to 90% by weight relative to the total weight of the composition;
b) an aqueous phase in a proportion of from 50 to 98.9% by weight relative to the total weight of the composition; and
c) at least one emulsifier in a proportion of from 1 to 10% by weight relative to the total weight of the composition.

20. The method of Claim 19, wherein the fatty substance is selected from the group consisting of isododecane, hydrogenated polyisobutene, squalane, isononyl isononanoate, cyclotetra- and - pentadimethicones, phenyltrimethicone, ethylene homopolymers, ethoxylated fats and oils, fluoroalkanes, microcrystalline waxes, ozocerite, beeswax, seracite, shea butter, candelilla wax, arachidyl propionate, fluoropolymers represented by the monomer:
X₁X₂C=CX₃F
wherein X₁, X₂, and X₃ are independently H or F, and copolymers of ethylene and of at least one monomer represented by the formula:
CH₂ =CH-R₃
wherein R₃ is an alkyl radical containing from 1 to 30 carbon atoms or an aryl or aralkyl radical.

21. The method of Claim 1 wherein the composition is selected from the group consisting of skin care products, skin cleansing products, make-up, facial lotions, crème moisturizers, body washes, body lotions, foot and hand crèmes, lipsticks, eyeshadows, foundations, shaving crème compositions, shaving lotions, crème depilatories, lotion depilatories, facial masks and anti-aging products.

22. The method of any of Claims 1 to 21 wherein this method is a cosmetic method.

23. The use of a composition as defined in any of claims 1 to 21 for bringing about a tightening effect of skin (preferably human skin).

## Patentansprüche

1. Ein Verfahren zur Herbeiführung eines Straffungseffektes von Haut (bevorzugt menschlicher Haut) umfassend das Applizieren einer Zusammensetzung, umfassend eine wirksame Menge eines Sojaproteins ausgewählt aus der Gruppe bestehend aus Sojaproteine, welche modifiziert wurden durch Behandlung mit einem Reduktionsmittel und anschließender Reaktion mit einem Carbonsäureanhydrid, Sojaproteine, welche modifiziert wurden durch Behandlung mit einem Reduktionsmittel, mit einem Carbonsäureanhydrid zur Reaktion gebracht wurden und anschließend oxidiert wurden, Sojaproteine, welche modifiziert wurden durch die Reaktion mit einem Hydroxyalkylacrylat, Sojaproteine, welche modifiziert wurden mit Epoxid, Acrylat oder ionischen Chlorhydrinmonomeren, Sojaproteine, welche modifiziert wurden durch Reaktion mit einem Alkylacrylimidoglycolat, und Mischungen dieser, auf die Haut.

2. Das Verfahren nach Anspruch 1 wobei besagte Zusammensetzung weiterhin umfasst mindestens ein kosmetisches Hilfsmittel ausgewählt aus der Gruppe bestehend aus Füllstoffen, Tensiden, Tixotropiermittel, Antioxidantien, Konservierungsstoffe, Farbstoffe, Pigmente, Duftstoffe, Verdicker, Vitamin, Hormone, Feuchthaltemittel, organische UVabsorbierende Sonnenschutzmittel, anorganische UV-streuende Sonnenschutzmittel, kationischen und anionischen und nicht-ionischen oder amphoteren Polymeren.

3. Das Verfahren nach Anspruch 1, wobei die wirksame Menge der Sojaproteine 0,001 bis 90 Gew.-% der Zusammensetzung beträgt.

4. Das Verfahren nach Anspruch 1, wobei die wirksame Menge der Sojaproteine von 0,001 bis 60 Gew.-% der Zusammensetzung beträgt.

5. Das Verfahren nach Anspruch 1, wobei die wirksame Menge der Sojaproteine von 0,005 bis 20 Gew.-% der Zusammensetzung beträgt.

6. Das Verfahren nach Anspruch 1, wobei die Zusammensetzung weiterhin umfasst eine wirksame Menge eines natürlichen oder rekombinanten Proteins oder eines hydrolysierten natürlichen oder rekombinanten Proteins.

7. Das Verfahren nach Anspruch 6, wobei die wirksame Menge des natürlichen oder rekombinanten Proteins von 0,01 bis 60 Gew.% der Zusammensetzung beträgt.

8. Das Verfahren nach Anspruch 6, wobei die wirksame Menge des natürlichen oder rekombinanten Proteins von 0,01 bis 20 Gew.-% der Zusammensetzung beträgt.

9. Das Verfahren nach Anspruch 6, wobei das natürliche oder rekombinante Protein ausgewählt ist aus der Gruppe bestehend aus Weizen-, Hafer- und Reisproteine, Kollagen, Keratine, Gelatine, Elastin, Fibronektin, lösliches Retikulin, unmodifiziertes Sojaproteine in unhydrolysierter oder hydrolysierter Form und wasserlösliche Seidenproteine.

10. Das Verfahren nach Anspruch 1, wobei die Zusammensetzung eine wasserfreie Zusammensetzung ist und 10 - 90 Gew.-%, relativ zum gesamten Gewicht der Zusammensetzung, einer Fettphase enthält, wobei die Fettphase zumindest eine flüssige, feste oder halbfeste fettige Substanz enthält.

11. Das Verfahren nach Anspruch 10, wobei die fettige Substanz ausgewählt ist aus der Gruppe bestehend aus Isododecan, hydriertes Polyisobuten, Squalen, Isononylisononanoat, Cyclotetra- und Pentadimeticone, Phenyltrimeticon, Ethylenhomopolymere, ethoxylierte Fette und Öle, Fluoralkane, microkristalline Wachse, Ozocerit, Bienenwachs, Ceracit, Sheabutter, Candellila Wachs, Arachidylpropionat, Fluorpolymere repräsentiert durch das Monomer:
X₁X₂C=CX₃F
wobei X₁, X₂ und X₃ unabhängig voneinander H oder F sind, und Copolymere des Ethylens und zumindest einem Monomer repräsentiert durch die Formel:
CH₂=CH-R₃
wobei R₃ ein Alkylradikal ist, welches von 1- 30 Kohlenstoffatome enthält, oder ein Aryl oder Aralkylradikal.

12. Das Verfahren nach Anspruch 1, wobei die Zusammensetzung in der Form einer wässrigen Lösung vorliegt, welche Mono- oder Polyalkohole enthält.

13. Das Verfahren nach Anspruch 1, wobei die Zusammensetzung vorliegt in der Form einer Cremeemulsion, eines Gels, eines trockenen Pulvers, eines Aerosols, eines Schaums, einer Alkohol-in-Öl Emulsion, einer Alkohol und Wasserlösung, einer wässrigen Lösung oder einer Emulsionslösung.

14. Das Verfahren nach Anspruch 13, wobei die Zusammensetzung weiterhin umfasst ein Carbomer, Gummi, oder ein anderer Verdicker in einer Menge von 0,1 bis 15 Gew.-%, bezogen auf das gesamte Gewicht der Zusammensetzung.

15. Das Verfahren nach Anspruch 13, wobei zumindest eines der Materialien in Form eines Pulvers vorliegt und wobei die wirksame Menge des Sojaproteins zusammen mit einem Pigment oder Füllstoff vorliegt.

16. Das Verfahren nach Anspruch 15, wobei die wirksame Menge des Sojaproteins von 0,001 bis 60 Gew.-% beträgt.

17. Das Verfahren nach Anspruch 15, wobei das Pigment ausgewählt ist aus der Gruppe bestehend aus Titandioxid, Zinkoxid, Bariumoxid, DC rot Nr. 36 und DC orange Nr. 17, Calciumlake von DC rot Nr. 7, 11, 31 und 34, Bariumlake von DC rot Nr. 12, Strontiumlake von DC rot Nr. 13, Aluminiumlaken von FD und C gelb Nr. 5, von FD gelb und C Nr. 6, von DC rot Nr. 27, von DC rot Nr. 21 und von FD und C blau Nr. 1, Eisenoxiden, Manganviolet, Chromoxid, Ultramarinblau und Rußpartikel.

18. Das Verfahren nach Anspruch 15, wobei der Füllstoff ausgewählt ist aus der Gruppe bestehend aus Calciumcarbonat, Aluminiumsilikat, Calciumsilikat, Magnesiumsilikat, Glimmer, Talkum, Bariumsulfat, Calciumsulfat, gepulverte Polyamide, gepulverte Polyester, gepulverte fluorierte Alkane und andere inerte gepulverte Kunststoffe.

19. Das Verfahren nach Anspruch 1, wobei die Zusammensetzung in Form einer stabilen Dispersion von Wasser-in-Öl oder Öl-in-Wasser Form vorliegt und umfasst:
a) eine Fettphase in einer Menge von 0,1 - 50 Gew.-% bezogen auf das gesamte Gewicht der Zusammensetzung, wobei die Fettphase das Sojaprotein in einer Menge von 0,001 - 50 Gew.-% bezogen auf das Gesamtgewicht der Zusammensetzung enthält;
b) eine wässrige Phase in einer Menge von 50 - 98,9 Gew.-% bezogen auf das gesamte Gewicht der Zusammensetzung; und
c) zumindest ein Emulgator in einer Menge von 1-10 Gew.-% bezogen auf das gesamte Gewicht der Zusammensetzung.

20. Das Verfahren nach Anspruch 19, wobei die fettige Substanz ausgewählt ist aus der Gruppe bestehend aus Isododecan, hydriertes Polyisobuten, Squalen, Isononylisononanoat, Cyclotetra- und Pentadimeticon, Phenyltrimeticon, Ethylenhomopolymere, ethoxylierten Fetten und Ölen, Fluoralkan, microkristallinen Wachsen, Ozocerit, Bienenwachs, Ceracit, Sheabutter, Candellila Wachs, Arachidylpropionat, Fluorpolymere repräsentiert durch das Monomer:
X₁X₂C=CX₃F
wobei X₁, X₂ und X₃ unabhängig voneinander H oder F sind und Copolymere des Ethylens und zumindest eines Monomers repräsentiert durch die Formel:
CH₂=CH-R₃
wobei R₃ ein Alkylradikal ist, welches 1-30 C-Atome enthält oder ein Aryl oder Aralkylradikal ist.

21. Das Verfahren nach Anspruch 1, wobei die Zusammensetzung ausgewählt ist aus der Gruppe bestehend aus Hautpflegeprodukten, Hautreinigungsprodukten, Make-up, Gesichtslotionen, Feuchtigkeitscremes, Körperwaschmittel, Körperlotionen, Fuß- und Handcreme, Lippenstiften, Lidschatten, Basismittel, Rasiercreme-Zusammensetzungen, Rasierlotionen, Enthaarungscremes, Enthaarungslotionen, Gesichtsmasken und Anti-ageing Produkten.

22. Das Verfahren nach einem der Ansprüche 1 bis 21, wobei dieses Verfahren ein kosmetisches Verfahren ist.

23. Die Verwendung einer Zusammensetzung wie definiert in einem der Ansprüche 1 bis 21 zur Herbeiführung eines Straffungseffektes von Haut (bevorzugt menschlicher Haut).

## Revendications

1. Méthode d'obtention d'un effet de resserrement de la peau (préférentiellement de la peau humaine), ladite méthode comprenant l'application à la peau d'une composition comprenant une quantité active d'une protéine de soja choisie dans le groupe constitué par les protéines de soja modifiées par un traitement par un agent réducteur et ayant ensuite réagi avec un anhydride d'acide carboxylique ; les protéines de soja modifiées par un traitement par un agent réducteur, ayant ensuite réagi avec un anhydride d'acide carboxylique, puis oxydées ; les protéines de soja modifiées par réaction avec un acrylate d'hydroxyalkyle ; les protéines de soja modifiées par des monomères ioniques de type époxyde, acrylate ou chlorhydrine ; les protéines de soja modifiées par réaction avec un acrylimidoglycolate d'alkyle ; et leurs mélanges.

2. Méthode conforme à la Revendication 1, où ladite composition comprend en outre au moins un adjuvant cosmétique choisi dans le groupe constitué par les charges, les tensioactifs, les agents thixotropes, les antioxydants, les conservateurs, les teintes, les pigments, les parfums, les épaississants, les vitamines, les hormones, les agents humidifiants, les écrans solaires organiques absorbant les UV, les écrans solaires inorganiques diffusant les UV, les agents mouillants, les polymères cationiques, anioniques et nonioniques ou amphotères.

3. Méthode conforme à la Revendication 1 où la quantité active de protéine de soja est comprise entre 0,001 et 90 % en masse de la masse totale de la composition.

4. Méthode conforme à la Revendication 1 où la quantité active de protéine de soja est comprise entre 0,001 et 60 % en masse de la masse totale de la composition.

5. Méthode conforme à la Revendication 1 où la quantité active de protéine de soja est comprise entre 0,005 et 20 % en masse de la masse totale de la composition.

6. Méthode conforme à la Revendication 1, où la composition comprend en outre une quantité active d'une protéine naturelle ou recombinante, ou d'une protéine hydrolysée naturelle ou recombinante.

7. Méthode conforme à la Revendication 6 où la quantité active de protéine naturelle ou recombinante est comprise entre 0,001 et 60 % en masse de la masse totale de la composition.

8. Méthode conforme à la Revendication 6 où la quantité active de protéine naturelle ou recombinante est comprise entre 0,01 et 20 % en masse de la masse totale de la composition.

9. Méthode conforme à la Revendication 6, où la protéine naturelle ou recombinante est choisie dans le groupe constitué par les protéines de blé, d'avoine et de riz, le collagène, les kératines, la gélatine, l'élastine, la fibronectine, la réticuline soluble, les protéines de soja non modifiées sous forme hydrolysée ou non, et les protéines de soie hydrosolubles.

10. Méthode conforme à la Revendication 1, où la composition est une composition anhydre qui contient entre 10 et 90 % en masse, par rapport à la masse totale de la composition, d'une phase grasse, la phase grasse contenant au moins une substance grasse liquide, solide ou semi-solide.

11. Méthode conforme à la Revendication 10, où la substance grasse est choisie dans le groupe constitué par l'isododécane, le polyisobutène hydrogéné, le squalane, l'isononanoate d'isononyle, la cyclotétra- et la pentadiméthicone, la phényltriméthicone, les homopolymères d'éthylène, les graisses et huiles éthoxylées, les fluoroalcanes, les cires microcristallines, l'ozocérite, la cire d'abeille, la séricite, le beurre de karité, la cire de candelilla, le propionate d'arachidyle, les fluoropolymères dont le monomère est :
X₁X₂C=CX₃F
où X₁, X₂ et X₃ représentent indépendamment H ou F, et les copolymères d'éthylène et d'au moins un monomère de formule :
CH₂=CH-R₃
où R₃ représente un radical alkyle comportant entre 1 et 30 atomes de carbone ou un radical aryle ou arylalkyle.

12. Méthode conforme à la Revendication 1, où la composition et sous forme d'une solution aqueuse contenant des monoalcools ou des polyols.

13. Méthode conforme à la Revendication 1, où la composition est sous forme d'une émulsion de type crème, d'un gel, d'une poudre sèche, d'un aérosol, d'une mousse, d'une émulsion alcool dans l'huile, d'une solution hydroalcoolique, d'une solution aqueuse ou d'une solution en émulsion.

14. Méthode conforme à la Revendication 13 où la composition comprend en outre un agent épaississant tel qu'un carbomère ou une gomme dans une proportion comprise 0,1 et 15 % en masse par rapport à la masse totale de la composition.

15. Méthode conforme à la Revendication 13 où au moins l'une des matières est sous la forme d'une poudre et où la quantité active de la protéine de soja est présente en même temps qu'un pigment ou une charge.

16. Méthode conforme à la Revendication 15 où la quantité active de protéine de soja est comprise entre 0,001 et 60 % en masse de la masse totale de la composition.

17. Méthode conforme à la Revendication 15, où le pigment est choisi dans le groupe constitué par le dioxyde de titane, l'oxyde de zinc, l'oxyde baryum, les colorants D&C Red No. 36 et D&C Orange No. 17, les qualités « Calcium Lake » des colorants D&C Red No. 7, 11, 31 and 34, la qualité « Barium Lake » du colorant D&C Red No. 12, la qualité « Strontium Lake » du colorant D&C Red No. 13, les qualités « Aluminum Lake » des colorants FD&C Yellow No. 5, FD&C Yellow No. 6, D&C Red No. 27, D&C Red No. 21 et FD&C Blue No. 1, les oxydes de fer, le violet de manganèse, l'oxyde de chrome, le bleu d'outremer et les particules de noir de carbone.

18. Méthode conforme à la Revendication 15 où la charge est choisie dans le groupe constitué par le carbonate de calcium, le silicate d'aluminium, le silicate de calcium, le silicate de magnésium, le mica, le talc, le sulfate de baryum, le sulfate de calcium, les polyamides en poudre, les polyesters en poudre, les alcanes fluorés en poudre et d'autres plastiques en poudre inertes.

19. Méthode conforme à la Revendication 1, où la composition et sous forme d'une dispersion stable d'eau dans l'huile ou d'huile dans l'eau, et comprend :
a) une phase grasse dans une proportion comprise entre 0,1 et 50 % en masse par rapport à la masse totale de la composition, la phase grasse contenant la protéine de soja dans une proportion comprise entre 0,001 et 90 % en masse par rapport à la masse totale de la composition ;
b) une phase aqueuse dans une proportion comprise entre 50 et 98,9 % en masse par rapport à la masse totale de la composition ; et
c) au moins un émulsifiant dans une proportion comprise entre 1 et 10 % en masse par rapport à la masse totale de la composition.

20. Méthode conforme à la Revendication 19, où la substance grasse est choisie dans le groupe constitué par l'isododécane, le polyisobutène hydrogéné, le squalane, l'isononanoate d'isononyle, la cyclotétra- et la pentadiméthicone, la phényltriméthicone, les homopolymères d'éthylène, les graisses et huiles éthoxylées, les fluoroalcanes, les cires microcristallines, l'ozocérite, la cire d'abeille, la séricite, le beurre de karité, la cire de candelilla, le propionate d'arachidyle, les fluoropolymères dont le monomère est :
X₁X₂C=CX₃F
où X₁, X₂ et X₃ représentent indépendamment H ou F, et les copolymères d'éthylène et d'au moins un monomère de formule :
CH₂=CH-R₃
où R₃ représente un radical alkyle comportant entre 1 et 30 atomes de carbone ou un radical aryle ou arylalkyle.

21. Méthode conforme à la Revendication 1, où la composition est choisie dans le groupe constitué par les produits de soin pour la peau, les produits purifiant la peau, le maquillage, les lotions pour le visage, les crèmes hydratantes, les savons corporels, les lotions corporelles, les crèmes pour les pieds et mains, les rouges à lèvres, les fards à paupières, les fonds de teint, les compositions de crème de rasage, les lotions après-rasage, les crèmes épilatoires, les lotions épilatoires, les masques pour le visage et les produits anti-âge.

22. Méthode conforme à l'une quelconque des Revendications 1 à 21, où cette méthode est une méthode cosmétique.

23. Emploi d'une composition définie dans l'une quelconque des revendications 1 à 21 pour l'obtention d'un effet de resserrement de la peau, préférentiellement de la peau humaine.
